# EUROPEAN PATENT APPLICATION

(11) **EP 1 295 618 A1**
(43) Date of publication of application: **26.03.2003**
(21) Application number: 02714456.7
(22) Date of filing: 04.04.2002
(51) Int. Cl.: A61M 1/14

(54) **ARTIFICIAL DIALYSIS**

(30) Priority: 10.04.2001 JP 2001112032
(71) Applicant: Teijin Seiki Co., Ltd., Tokyo 105-8628 (JP)
(72) Inventor: FUKUI, Kiyozumi, c/o Teijin Seiki CO., LTD., Minato-ku, Tokyo 105-8628 (JP); ENDO, Takuma, c/o Teijin Seiki CO., LTD., Minato-ku, Tokyo 105-8628 (JP)
(74) Representative: Grünecker, Kinkeldey, Stockmair & Schwanhäusser Anwaltssozietät
(86) International application number: JP0203376
(87) International publication number: WO02083201

(57) **Abstract**

It is an object to solve problems incidental to the passage piping of a hemodialyzer, and more specifically to provide a hemodialyzer which is small-sized and inexpensive and has a great safety. As means for solving the problems, there is provided a hemodialyzer comprising dialytic basic devices such as a dialyzer, and fluid control devices such as a valve to be provided in a dialysis circuit, wherein a fluid passage block provided with a port for connecting the various devices is included and a fluid connecting circuit is formed between the ports in the block.

## Description

### Technical Field

The present invention relates to a hemodialyzer, and more specifically to a hemodialyzer which manifolds a dialysis fluid circuit to reduce the size and cost of the whole apparatus and to have a high durability and can be used at home.

### Related Art

A conventional hemodialyzer comprises a large number of components having various sizes, for example, dialytic basic devices such as a dialyzer, a pyrogen filter, a dialysate tank and a pump, and fluid control devices such as a valve, a pressure gauge and a flowmeter which are provided in a dialysis fluid circuit, and these devices are connected to each other through a pipe.

In such a conventional apparatus, however, even if the costs of various devices constituting the conventional apparatus are reduced, a large number of various piping components and a considerable working man-hour are required for the pipe connection of the devices. For this reason, the conventional apparatus is expensive and there is a high possibility that piping errors in manufacture might be made or functions might be deteriorated earlier after the start of use. Accordingly, there is a problem in that it is actually hard for an individual patient to purchase the apparatus and to carry out a dialysis at home or a considerable economical burden is imposed.

Furthermore, the conventional apparatus requires a space for the pipe connection. Consequently, there is a problem in that the whole apparatus becomes large-sized and is hard to move and to install in the home of a patient.

Moreover, a nonmetal tube such as a synthetic resin pipe is generally used for the pipe. Therefore, there is a possibility that a failure might be caused by a crack due to a deterioration.

The invention solves the problems incidental to the passage piping of the hemodialyzer and has an object to provide a hemodialyzer which is small-sized and inexpensive and has a great safety, thereby implementing a dialysis at home.

### Summary of the Invention

In the invention, the object is achieved by a hemodialyzer comprising dialytic basic devices such as a dialyzer, a pyrogen filter and a dialysate tank, and fluid control devices such as a valve, a flowmeter, a pressure gauge and a thermometer which are provided in a dialysis circuit, wherein a fluid passage block provided with a port for connecting the various devices is included and a fluid connecting circuit is formed between the ports in the block.

According to the invention, the fluid passage block provided with the port for connecting various fluid devices of the hemodialyzer is disposed and the fluid connecting circuit is formed between the ports in the block. Therefore, a piping work can be relieved considerably and a space for the piping is also decreased greatly. Consequently, it is possible to provide an inexpensive and small-sized hemodialyzer.

Furthermore, the piping is greatly decreased by manifolding the passage (the fluid passage block). Therefore, it is possible to decrease failures caused by the crack of a pipe, the attachment incompleteness of a connecting portion or the looseness of a screw portion generated by a heat cycle of a rise or fall in a temperature or a probability thereof as in the conventional art. Thus, it is possible to obtain an apparatus having a great safety.

Moreover, the fluid passage block is constituted by a plurality of plate members, each of the plate members is provided with a groove and a hole, and the plate members are superposed to constitute the fluid connecting circuit. Consequently, it is possible to inexpensively fabricate the block. Furthermore, if the block is fabricated by using a photomolding method, the complicated passage and the device connecting port can be molded completely and integrally. Thus, it is possible to obtain a manufacturing method which can attain the object of the invention still more.

As a result of these features, the hemodialyzer is strong and can easily be used by a user. Consequently, an excessive burden is not imposed and a dialysis can be implemented at home.

### Brief Description of the Drawings

Fig. 1 is a schematic conceptional view showing a hemodialyzer according to an embodiment of the invention.
Fig. 2 is a conceptional view showing the passage formation surface of a plate member constituting a fluid passage block in Fig. 1.
Fig. 3 is a conceptional view showing the partial section of the fluid passage block in Fig. 1.

### Embodiment of the Invention

An embodiment of the invention will be described below with reference to the drawings.

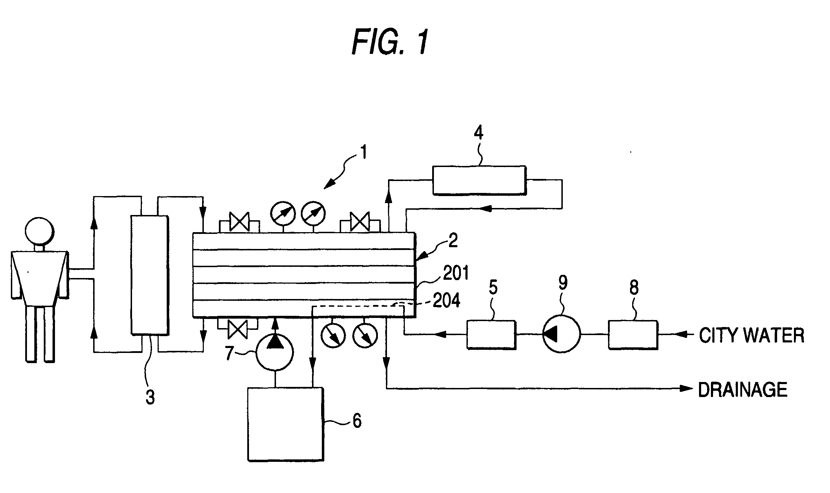

Fig. 1 is a schematic conceptional view showing a hemodialyzer 1 according to the embodiment of the invention. As shown in the drawing, the hemodialyzer 1 has various dialytic basic devices connected to a fluid passage block 2 which will be described below. Herein, a dialyzer 3, a pyrogen filter 4, an RO film 5, a dialysate tank 6, and a dialysate supply pump 7 which have comparatively large volumes are shown as basic devices to be connected to the block 2. Since these devices have large volumes, it is hard to directly attach them to the block 2 which will be described below. Therefore, the devices are connected to the block 2 through external piping as shown. In the invention, it is a matter of course that the dialyzer 3 may be directly attached to the block 2 without using such external piping.

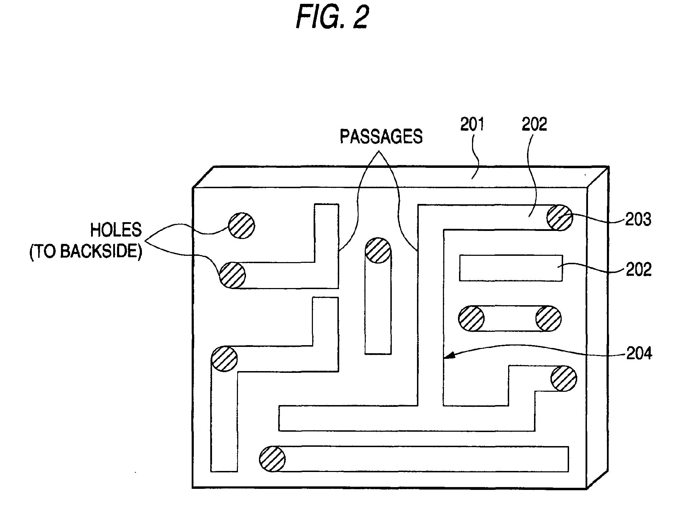

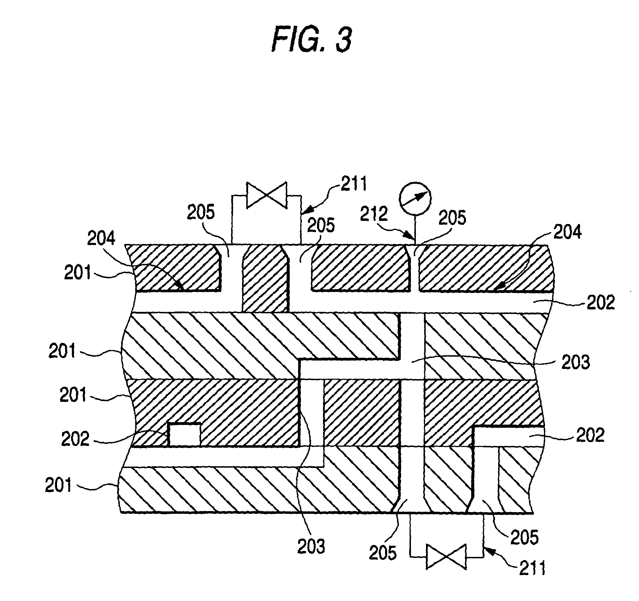

The fluid passage block 2 is constituted by a plurality of plate members 201 as shown in Fig. 1. As shown in Figs. 2 and 3 to be conceptional views illustrating the formation. of a fluid passage, each plate member 201 is provided with a groove 202 and a hole 203 by machining. The plate members 201 are superposed to form a fluid passage 204. The external wall portion of the fluid passage block 2 is provided with an attachment hole 205 for gasket mounting various control valves 211 and sensors (a pressure gauge or a flowmeter) 212 as a fluid control device to be provided in a dialytic circuit. The plate members 201 are integrated on a boundary surface thereof by fastening means with a bolt through a packing or a leakage preventing resin. Even if a fluid circuit in the fluid passage block 2 is complicated, a plurality of plate members 201 are combined to form the passage 204 so that a best fluid circuit can be designed.

Next, the passage 204 to be formed in the fluid passage block 2 and the device to be connected to the block 2 will be described with the explanation of the operation of the hemodialyzer 1.

As shown in Fig. 1, water from a water supply is fed to the fluid passage block 2 through a filter 8., an RO pump 9 and an RO film 5. The filter 8 serves to remove the impurity of the water. The water getting out of the filter 8 is pressurized by means of the RO pump 9 and is caused to pass through the RO film 5 to be purified water, and is fed to the block 2 through external piping. The purified water fed to the block 2 passes through the passage 204 in the block 2 and-is thus guided to the dialysate tank 6. Moreover, there is also a method of feeding a dialysate without the RO film 5 and the dialysate tank 6. At this time, an ultraviolet sterilizing device (not shown) for sterilizing the purified water is attached to an introducing portion to the block 2 or an introducing portion from the block 2 to the dialysate tank 6. In the tank 6, the purified water and an agent are mixed with each other so that a dialysate is prepared. The dialysate is guided to the block 2, and furthermore, passes through the fluid passage 204 in the block 2 and is thus guided to the pyrogen filter 4 for removing a heat generating material. The dialysate passing through the pyrogen filter 4 is guided into the block 2 again and is delivered to the dialyzer 3 through the passage 204 in the block 2. The dialysate passing through the dialyzer 3 is returned into the block 2 again and is then returned to the dialysate tank 6 through the passage 204 in the block 2, and circulates in the same cycle again.

For easy understanding, the above description has been generally given to a fluid circuit. Actually, various control circuits and safety circuits related to the valve 211 and the sensor 212 are incorporated in the fluid passage block 2. These circuits are formed by the passage 204 in the block 2 and the control valve 211 and the sensor 212 are connected to the passage 204 through the attachment hole 205 provided in the block 2. Moreover, other devices which are not shown comparatively large such as a blood pump are provided on the fluid passage block 2 or separately from the block 2, and their necessary connection to the passage 204 in the block 2 is carried out. The fluid passage block 2 subjected to manifolding may be properly divided into a plurality of parts depending on a design or piping. For example, the fluid passage block 2 may be divided into three blocks, for example, a blood circuit block portion directly connecting the dialyzer 3 to constitute a blood circuit, a dialysate feeding circuit block portion directly connecting the pyrogen filter 3 to constitute a dialysate feeding circuit and a water processing block portion directly connecting the RO film 5 to constitute a water supply processing circuit, and necessary external piping may be carried out between the blocks.

### Industrial Applicability

The hemodialyzer according to the invention has a main feature that the fluid passage is formed in the fluid passage block. Consequently, it is possible to wholly reduce a size, to more decrease the number of assembly components than that of the conventional apparatus having piping using a tube, to reduce a danger that a mechanical failure such as the leakage of a fluid might be caused, and to greatly reduce errors, for example, a complicated piping assembly is mistaken due to a large number of components. Furthermore, maintenance can efficiently be carried out after use because of the simplification of the piping, and a manufacturing cost and a maintenance cost for use can also be reduced greatly. Accordingly, it is possible to manufacture a hemodialyzer for home (an individual) which is inexpensive and has a great safety, and it is also possible to decrease the burden of a patient that has taken a long time to go to medical facilities for a hemodialysis at a remote place.

## Claims

1. A hemodialyzer comprising dialytic basic devices including a dialyzer, a pyrogen filter and a dialysate tank, and fluid control devices including a valve, a flowmeter and a pressure gauge which are provided in a dialysis circuit,
wherein the hemodialyzer further comprises a fluid passage block provided with ports for connecting said devices and a fluid connecting circuit formed inside the block so as to communicate the ports.

2. The hemodialyzer according to claim 1, wherein the fluid passage block is constituted by a plurality of plate members, each of the plate members is provided with a groove and a hole, and the plate members are superposed to constitute the fluid connecting circuit.

3. The hemodialyzer according to claim 1, wherein the fluid passage block is molded by a photomolding method.
